Europäisches Patentamt

European Patent Office

Office européen des brevets

(19)

(11) Numéro de publication: **0 181 798 B1**

(12) **FASCICULE DE BREVET EUROPEEN**

(45) Date de publication du fascicule du brevet:
03.05.89

(51) Int. Cl.4: **G 01 N 33/553, B 03 C 1/28, H 01 F 7/20, B 25 B 11/00**

(21) Numéro de dépôt: **85402029.4**

(22) Date de dépôt: **21.10.85**

(54) **Nouveau dispositif magnétique destiné à retirer des billes de gel magnétique d'un milieu à analyser et à les transférer sur un milieu de dosage immunoenzymatique.**

(30) Priorité: **23.10.84 FR 8416170**

(43) Date de publication de la demande:
**21.05.86 Bulletin 86/21**

(45) Mention de la délivrance du brevet:
**03.05.89 Bulletin 89/18**

(84) Etats contractants désignés:
**AT BE CH DE FR GB IT LI LU NL SE**

(56) Documents cités:
**EP-A- 0 140 787**
**CH-A- 233 674**
**US-A- 1 904 224**
**US-A- 2 471 764**
**US-A- 3 985 649**
**US-A- 4 272 510**

(73) Titulaire: **INSTITUT PASTEUR Fondation reconnue d'utilité publique, 28 rue du Docteur Roux, F-75724 Paris Cédex 15 (FR)**

(72) Inventeur: **Dodin, André, Courances, F-91490 Milly-la-Forêt (FR)**
Inventeur: **Muller, Bernard, 5, rue Raymond Vidal, F-78100 St-Germain-en-Laye (FR)**

(74) Mandataire: **Orès, Bernard et al, Cabinet ORES 6, Avenue de Messine, F-75008 Paris (FR)**

ACTORUM AG

## Description

La présente invention est relative à un nouveau dispositif magnétique destiné à retirer des billes de gel magnétique d'un milieu liquide, biologique ou non, tel que le sang, l'urine, l'eau, le lait, les effluents, etc. . ., pour permettre de réaliser un diagnostic de germes pathogènes en transférant ces billes sur un milieu de dosage immunoenzymatique.

Le Brevet au nom de l'INSTITUT PASTEUR FR-A-2 554 016 décrit un dispositif du type comportant une tige aimantée, dans lequel la tige est constituée d'une partie supérieure en matériau non magnétique emboîtée, à l'aide d'un cliquet ou système à baïonnette, dans une partie inférieure en matériau magnétique.

Cette tige coopère avec un boîtier en matériau isolant, notamment réalisé sous forme de poignée isolante, qui contient un aimant et est pourvu sur toute sa hauteur d'un orifice traversant, en particulier excentré, de guidage de la tige; ladite tige coopère aussi avec un ressort monté sur cette tige entre une rondelle de butée, fixée au niveau de l'extrémité supérieure de la tige, et la face supérieure du boîtier: une pression exercée sur l'extrémité supérieure de la tige provoque le coulissement de cette dernière vers le bas pour retirer des billes de gel magnétique d'un milieu à analyser, tandis que le relâchement de ladite pression provoque le rappel de la tige sous l'action du ressort et permet de transférer lesdites billes magnétiques sur un milieu de dosage immunoenzymatique de manière à réduire sensiblement, au cours de l'opération de transfert, tout contact accidentel indésirable entre la tige aimantée et des surfaces naturellement magnétiques, en mettant ainsi en œuvre les procédés de dosage objets des Brevets au nom de l'INSTITUT PASTEUR FR-A-2 334 106 et FR-A-2 537 725.

On connaît aussi le Brevet US-A-4 272 510 (SMITH et al.) qui décrit un procédé utilisable dans le cadre des dosages immunoradiologiques, ou analogues, pour transférer une pluralité d'unités antigène/anticorps, notamment à l'aide de particules sous forme de billes magnétiques appropriées, par attraction magnétique à l'aide d'un dispositif de transfert approprié pourvu d'une pluralité de sondes en forme de tiges en matériau magnétique destinées à être immergées dans une pluralité de réceptacles contenant un milieu liquide à analyser.

Parmi les différents dispositifs de transfert proposés par SMITH et al., le Brevet américain en question en décrit un qui comporte une pluralité de sondes électromagnétiques, chacune constituée par une tige en matériau magnétique dont la portion supérieure est entourée par un enroulement de spires de fil métallique isolé (émaillé) inséré dans un circuit d'excitation comportant une source d'alimentation en courant continu, notamment constituée par une pile électrique, et un interrupteur. Cette pluralité de sondes électromagnétiques est portée par un boîtier approprié duquel font saillie vers l'extérieur et vers le bas les tiges de façon à les immerger dans le milieu liquide à analyser.

La présente invention s'est donné pour but de pourvoir à un dispositif magnétique qui répond mieux aux nécessités de la pratique que les dispositifs magnétiques susdits visant au même but, notamment en ce que:

– l'on élimine la nécessité de disposer un aimant auxiliaire à l'extérieur du récipient contenant le milieu de dosage immunoenzymatique, et

– le dispositif est d'une plus grande maniabilité.

La présente invention a pour objet un dispositif magnétique destiné à retirer des billes de gel magnétique ou analogues d'un milieu liquide à analyser, biologique ou non, et à les tranférer sur un milieu de dosage immunoenzymatique, lequel dispositif est du type comportant:

– une tige en un matériau ferromagnétique, notamment en fer doux, de longueur appropriée,

– un boîtier, de préférence oblong, réalisé en un matériau non magnétique, en particulier en matière plastique,

– un circuit d'excitation électromagnétique de la tige comportant à son tour:

– un enroulement électrique d'excitation, logé dans ledit boîtier et enroulé autour de la tige,

– un noyau discontinu en matériau ferromagnétique constitué par la même tige dont la portion supérieure est enveloppée par le boîtier, tandis que la portion inférieure se projette à l'extérieur du boîtier,

– une source d'alimentation du circuit d'excitation, notamment constituée par une pile électrique,

– un contacteur de commande de ce circuit d'excitation, lequel dispositif est caractérisé en ce qu'il est pourvu de moyens de fixation et de centrage de la tige par serrage.

Selon un mode de réalisation avantageux du dispositif conforme à l'invention, les moyens de fixation et de centrage de la tige par serrage sont constitués par un manchon déformable, notamment en caoutchouc, et par un manchon rigide de pression de ce manchon déformable, lesquels manchons sont disposés autour de la tige et coopèrent avec un moyen de logement, dans lequel ils sont disposés bout à bout, ainsi qu'avec un moyen de compression du manchon rigide contre le manchon déformable, dont la déformation permet de serrer la tige et donc de la fixer et centrer par rapport au boîtier.

Selon une disposition avantageuse de ce mode de réalisation, le moyen de logement des manchons déformable et rigide est constitué par un manchon coaxial avec le boîtier et réalisé en une seule pièce par moulage avec ce dernier, lequel manchon de logement fait saillie vers l'extérieur et vers le bas à partir de la base de fermeture du boîtier qui est percée d'un orifice central permettant le passage de la tige, et en ce que le moyen de compression du manchon rigide contre le manchon déformable est constitué par une bague en matière plastique se vissant autour du manchon de logement.

Selon un autre mode de réalisation avantageux du dispositif conforme à l'invention, la pile d'alimentation est logée dans le boîtier, où elle est disposée au-dessus de l'enroulement d'excitation qui est en forme de solénoïde et repose sur la base de fermeture du boîtier, et en ce que ce boîtier comporte un capot de fermeture, également en matériau non magnétique, qui se visse autour de l'extrémité supérieure du boîtier et qui est pourvu d'un orifice central permettant le montage de l'interrupteur du circuit d'excitation constitué par un contacteur de commande.

Selon une autre disposition avantageuse de ce mode de réalisation, le solénoïde d'excitation est enroulé directement sur la tige en fer doux.

Selon une variante préférée de cette disposition, le solénoïde d'excitation est enroulé autour de la tige par l'intermédiaire d'un support solide, notamment constitué par un manchon en matériau non magnétique, qui est emmanché légèrement à force autour de la tige.

Selon un autre mode de réalisation avantageux du dispositif conforme à l'invention, la tige est amovible et stérilisable.

Selon un autre mode de réalisation avantageux du dispositif conforme à l'invention, la tige est interchangeable en fonction de son diamètre.

Selon une disposition préférée de ce mode de réalisation la tige comporte une portion supérieure de diamètre constant, destinée à être enveloppée par ledit solénoïde et ledit manchon de logement, et une portion inférieure de diamètre variable en fonction sensiblement inverse de la concentration prévisible de germes pathogènes du milieu à analyser.

L'invention sera mieux comprise à l'aide du complément de description qui va suivre, qui se réfère aux dessins annexés dans lesquels:

— la figure 1 est une vue en élévation d'ensemble du dispositif magnétique conforme à la présente invention, et

— la figure 2 est une vue en coupe longitudinale du dispositif représenté à la figure 1.

Il doit être bien entendu, toutefois, que ces dessins et les parties descriptives correspondantes, sont donnés uniquement à titre d'illustration de l'objet de l'invention, dont ils ne constituent en aucune manière une limitation.

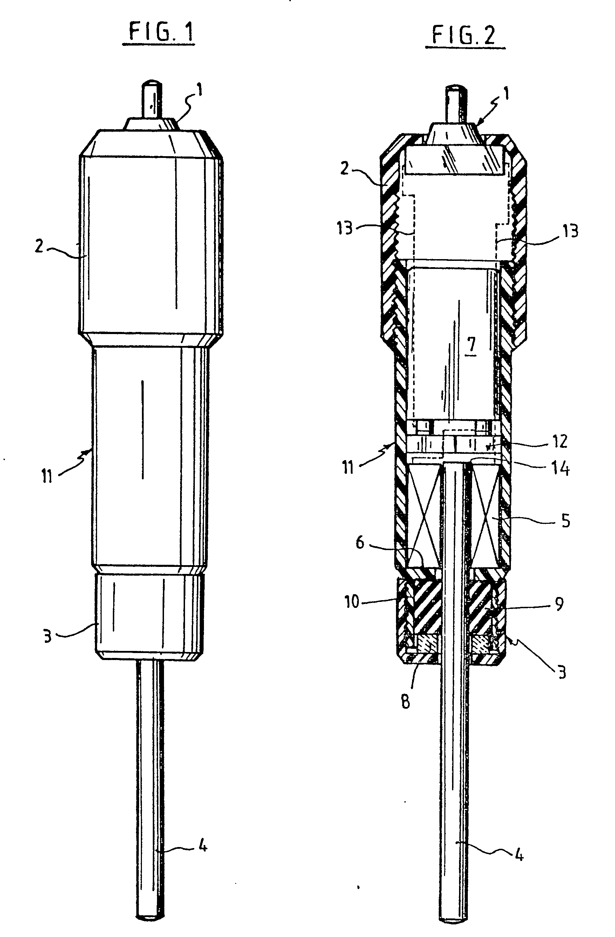

La figure 1 représente le dispositif magnétique selon l'invention en position de repos, à savoir avec le contacteur de commande 1 non enfoncé ou ouvert.

On peut distinguer, en allant du haut vers le bas, outre ledit contacteur 1, un capot 2 auquel ce dernier est fixé et qui enveloppe l'extrémité supérieure d'un boîtier 11 oblong de préférence cylindrique, et une bague 3 à la partie inférieure du boîtier 11: ce dernier, de même que le capot 2 et la bague 3, sont réalisés en matériau isolant, notamment en matière plastique.

Une tige métallique 4, notamment réalisée en fer doux, fait saillie vers le bas à partir du centre de la bague 3.

La figure 2 met en évidence les relations mutuelles des différents éléments qui entrent dans la composition du dispositif selon l'invention, quand ils sont assemblés.

En particulier, on peut distinguer les composants suivants du circuit d'excitation électromagnétique de la tige 4, à savoir:

— un solénoïde d'excitation 5, qui est réalisé avec un nombre de spires approprié (environ 350 spires de fil émaillé de diamètre égal à 0,5 mm dans l'exemple représenté) et qui est disposé à l'intérieur dudit boîtier 11 de manière à venir en appui sur la base 6 de fermeture de ce boîtier, et

— une pile 7 d'alimentation en courant continu du circuit d'excitation électromagnétique, qui est en particulier du type rechargeable et qui est disposée au-dessus du solénoïde 5; la pile est séparée de ce dernier par un dispositif à clip 12, connu en soi, aux bornes duquel sont raccordées une borne du contacteur 1 et une borne du solénoïde 5, respectivement: ainsi la pile 7, le contacteur 1 et le solénoïde 5 sont en série l'un par rapport à l'autre et constituent la partie électrique du circuit électromagnétique d'excitation de la tige 4 (dans l'exemple en question, on utilise une pile de 9 volts). La référence 13 désigne les fils de raccordement.

La partie magnétique (ou noyau) de ce circuit d'excitation électromagnétique est constituée par la même tige en fer doux 4, autour de laquelle est enroulé le solénoïde 5. Cette tige passe à travers un orifice ménagé dans la bague 3, ainsi que dans une rondelle 8 en «Teflon» (PTFE) et dans un joint de caoutchouc 9 logés à l'intérieur d'un manchon 10: il est avantageux que la hauteur du solénoïde 5 soit au minimum égale à $1/4$ de la longueur de la tige 4 qui, dans l'exemple représenté, est de 130 mm.

Le solénoïde 5 peut être soit directement enroulé sur la tige en fer doux 4, mais dans ce cas la tige ne peut être stérilisée par autoclavage ou par mise en contact avec un liquide désinfectant, soit de préférence ledit solénoïde peut être enroulé sur un support solide, par exemple constitué par un manchon 14, et dans ce cas la tige en fer, n'étant pas solidaire du support du solénoïde, peut être stérilisée en raison de sa mobilité.

Ce manchon, qui est coaxial avec le boîtier 11, fait saillie vers le bas à partir de la base 6 de ce boîtier et est pourvu à sa surface extérieure d'un filetage permettant le vissage de ladite bague 3: étant donné qu'avant le vissage, ladite rondelle 8 dépasse légèrement à l'extérieur du manchon 10, la compression de cette rondelle 8 contre le joint 9 au moment du vissage se transmet à ce dernier qui, par dilatation radiale, est capable de serrer la tige 4 et de la maintenir en place tout en assurant l'étanchéité nécessaire par rapport au milieu à analyser, au milieu de dosage et au liquide de rinçage. En fait, après chaque utilisation du dispositif selon l'invention, on effectue un rinçage de la tige avec de l'eau physiologiquement stérile, suivi par un passage à l'alcool et par un flambage: la protection du joint 9 de caoutchouc vis-à-vis de la chaleur développée par la flamme est assurée par la rondelle en «Teflon» 8.

Le système de maintien en position de la tige 4, tel qu'il vient d'être décrit, est particulièrement avantageux lorsque la tige est amovible, parce que – nous le répétons – cette disposition permet sa stérilisation en autoclave, notamment avant la première utilisation de la tige (lorsqu'on travaille avec un même milieu, il est en général suffisant de passer à la flamme la tige, après rinçage et passage à l'alcool).

Un deuxième avantage lié au fait que la tige est rendue amovible, est constitué par la possibilité de la rendre également interchangeable, notamment en fonction de son diamètre. A cet effet, il y a lieu de remarquer que pour les milieux à analyser présentant de faibles concentrations de germes pathogènes, le diamètre préférentiel de la tige 4 est égal à 8 mm, tandis que pour les concentrations moyennes et hautes, on préfère utiliser des tiges dont les diamètres sont égaux à 5 et 3 mm, respectivement.

Dans cet ordre d'idées, pour ne pas être obligés de changer notamment également le solénoïde et le joint de serrage étanche de la tige, pour adapter leurs orifices traversants à chacune des valeurs préférentielles de diamètre indiquées plus haut pour cette tige, il est avantageux que la tige 4 comporte une portion supérieure correspondant, en particulier, au diamètre le plus grand et destinée à être enveloppée par ledit solénoïde et ledit manchon 10, et une portion inférieure (non représentée) correspondant sensiblement à la partie qui dépasse de la bague de serrage 8, dont le diamètre est variable en fonction inverse de la concentration prévisible de germes pathogènes, suivant les considérations développées précédemment. Il va de soi que, lorsqu'il n'est pas possible de prévoir dans quelle gamme de valeurs (hautes, moyennes ou faibles) se trouve l'ordre de grandeur des concentrations des milieux à analyser, l'emploi de la tige de plus grand diamètre s'adapte à chaque cas.

Le fonctionnement du dispositif de prélèvement de billes magnétiques selon la présente invention est simple: une pression dudit contacteur de commande, notamment à l'aide du pouce de l'opérateur, permet d'exciter le circuit électro-magnétique duquel fait partie la tige de prélèvement, tandis que le relâchement de ce contacteur annule le champ magnétique initialement maintenu par la circulation du courant électrique à travers le solénoïde.

Dans son application aux procédés de dosage immunoenzymatique qui constituent l'objet des Brevets précités FR-A-2 334 106 et FR-A-2 537 725, le dispositif de prélèvement conforme à l'invention est placé au-dessus d'un récipient contenant un milieu d'incubation qui comprend un milieu liquide, biologique ou non, et des billes de gel magnétique couplées à une protéine appropriée permettant de réaliser le dosage immunoenzymatique: il suffit d'introduire la pointe de la tige de prélèvement, pendant que l'on appuie sur le contacteur, et de transférer les billes de gel magnétique qui ont été attirées par la tige et qui se sont fixées à la pointe de cette dernière, sur la surface d'un milieu de dosage immunoenzymatique approprié contenu dans un autre récipient, en relâchant le contacteur de commande: de cette manière, une partie au moins des billes prélevées se sépare par adhésion au milieu de dosage, en sorte qu'aucun aimant auxiliaire n'est plus nécessaire. (Il y a lieu de rappeler qu'avec le dispositif de prélèvement objet du Brevet évoqué plus haut FR-A-2 554 016, à cause de la forte aimantation résiduelle induite par l'aimant permanent de prélèvement dans la partie inférieure magnétique de la tige, il faut utiliser un aimant permanent plus puissant que l'aimant de prélèvement et, qui est disposé à l'extérieur du récipient contenant le milieu de dosage, pour séparer les billes de gel magnétique prélevées de la tige qui les porte).

Après avoir déposé sur le milieu de dosage les billes magnétiques, on rince la tige avec de l'eau physiologiquement stérile, pour la passer ensuite à l'alcool et la soumettre au flambage – comme indiqué plus haut – de manière à ce que le dispositif conforme à l'invention soit prêt pour une nouvelle opération.

**Revendications**

1. Dispositif magnétique destiné à retirer des billes de gel magnétique ou analogues d'un milieu liquide à analyser, biologique ou non, et à les transférer dans un milieu de dosage immunoenzymatique, lequel dispositif est du type comportant:
   – une tige (4) en un matériau ferromagnétique, notamment en fer doux, de longueur appropriée,
   – un boîtier (11), de préférence oblong, réalisé en un matériau non magnétique, en particulier en matière plastique,
   – un circuit d'excitation électromagnétique (1-5-7-13) de la tige (4) comportant à son tour:
   – un enroulement électrique d'excitation, logé dans ledit boîtier (11) et enroulé autour de la tige (4),
   – un noyau discontinu en matériau ferromagnétique constitué par la même tige (4) dont la portion supérieure est enveloppée par le boîtier, tandis que la portion inférieure se projette à l'extérieur du boîtier,
   – une source d'alimentation du circuit d'excitation, notamment constituée par une pile électrique,
   – un contacteur (1) de commande de ce circuit d'excitation,
   lequel dispositif est caractérisé en ce qu'il est pourvu de moyens de fixation et de centrage de la tige (4) par serrage.

2. Dispositif selon la revendication 1, caractérisé en ce que les moyens de fixation et de centrage de la tige (4) par serrage sont constitués par un manchon déformable (9), notamment en caoutchouc, et par un manchon rigide (8) de pression de ce manchon déformable, lesquels manchons sont disposés autour de la tige et coopèrent avec un moyen de logement, dans lequel ils sont disposés bout à bout, ainsi qu'avec un moyen de compression du manchon rigide (8) contre le manchon déformable (9) dont la déformation permet de serrer la tige (4) et donc de la fixer et centrer par rapport au boîtier (11).

3. Dispositif selon la revendication 2, caractérisé en ce que le moyen de logement des manchons déformable (9) et rigide (8) est constitué par un manchon (10) coaxial avec le boîtier (11) et réalisé en une seule pièce par moulage avec ce dernier, lequel manchon de logement (10) fait saillie vers l'extérieur et vers le bas à partir de la base (6) de fermeture du boîtier qui est percée d'un orifice central permettant le passage de la tige (4), et en ce que le moyen de compression du manchon rigide (8) contre le manchon déformable (9) est constitué par une bague (3) en matière plastique se vissant autour du manchon de logement (10).

4. Dispositif selon l'une quelconque des revendications 1 à 3, caractérisé en ce que la pile d'alimentation (7) est logée dans le boîtier (11), où elle est disposée au-dessus de l'enroulement d'excitation qui est en forme de solénoïde (5) et repose sur la base (6) de fermeture du boîtier, et en ce que ce boîtier (11) comporte un capot de fermeture (2), également en matériau non magnétique, qui se visse autour de l'extrémité supérieure du boîtier et qui est pourvu d'un orifice central permettant le montage de l'interrupteur du circuit d'excitation constitué par un contacteur de commande (1).

5. Dispositif selon l'une quelconque des revendications 1 à 4, caractérisé en ce que la tige (4) est amovible et stérilisable, notamment par autoclavage.

6. Dispositif selon l'une quelconque des revendications 1 à 5, caractérisé en ce que la tige (4) est interchangeable en fonction de son diamètre.

7. Dispositif selon la revendication 6, caractérisé en ce que la tige (4) comporte une portion supérieure de diamètre constant, destinée à être enveloppée par ledit solénoïde (5) et ledit manchon de logement (10), et une portion inférieure de diamètre variable en fonction inverse de la concentration prévisible de germes pathogènes du milieu à analyser.

8. Dispositif selon la revendication 4, caractérisé en ce que le solénoïde d'excitation (5) est enroulé directement sur la tige en fer doux (4).

9. Dispositif selon la revendication 4, caractérisé en ce que le solénoïde d'excitation (5) est enroulé autour de la tige (4) par l'intermédiaire d'un support solide, notamment constitué par un manchon (14) en matériau non magnétique, qui est emmanché légèrement à force autour de la tige (4).

**Patentansprüche**

1. Magnetische Einrichtung, die dazu bestimmt ist, magnetische Gelkugeln oder dergleichen aus einem zu analysierenden flüssigen Medium, das biologisch ist oder nicht, zu entnehmen und sie in ein immunoenzymatisches Dosiermedium zu überführen, welche Einrichtung von der Art ist, die enthält:
   – einen Schaft (4) aus einem ferromagnetischen Material, insbesondere aus Weicheisen, von geeigneter Länge,
   – ein Gehäuse (11), vorzugsweise länglich, das aus einem nichtmagnetischen Material, insbesondere aus Kunststoff, ausgeführt ist,
   – einen elektromagnetischen Erregerkreis (1-5-7-13) für den Schaft (4), der wiederum enthält:
   – eine elektrische Erregerwicklung, die in dem Gehäuse (11) untergebracht und um den Schaft (4) gewickelt ist,
   – einen diskontinuierlichen Kern aus ferromagnetischem Material, der von dem nämlichen Schaft (4) gebildet ist, dessen oberer Teil von dem Gehäuse ummantelt ist, während sich der untere Teil in das Äussere des Gehäuses erstreckt,
   – eine Betriebsstromversorgung für den Erregerkreis, die insbesondere von einem elektrischen Element gebildet ist,
   – einen Steuerschalter (1) für den Erregerkreis, welche Einrichtung dadurch gekennzeichnet ist, dass sie mit Mitteln zur Befestigung und zur Zentrierung des Schafts (4) durch Einspannung versehen ist.

2. Einrichtung nach Anspruch (1), dadurch gekennzeichnet, dass die Mittel zur Befestigung und zur Zentrierung des Schafts (4) durch Einspannung von einer deformierbaren Hülse (9), insbesondere aus Kautschuk, und von einer starren Druckhülse (8) für die deformierbare Hülse gebildet sind, welche Hülsen um den Schaft angeordnet sind und mit einem Aufnahmemittel zusammenwirken, in welchem sie Ende an Ende angeordnet sind, sowie mit einem Mittel zum Druck der starren Hülse (8) gegen die deformierbare Hülse (9), deren Deformation es ermöglicht, den Schaft (4) einzuspannen und mithin ihn mit Bezug auf das Gehäuse (11) zu befestigen und zu zentrieren.

3. Einrichtung nach Anspruch 2, dadurch gekennzeichnet, dass das Aufnahmemittel für die deformierbare (9) und starre (8) Hülse von einer Hülse (10) gebildet ist, die koaxial mit dem Gehäuse (11) und aus einem Stück mit letzterem durch Formen realisiert ist, welche Aufnahmehülse (10) nach dem Äusseren und nach unten von der Basis (6) des Abschlusses des Gehäuses, der mit einer mittigen Öffnung durchlocht ist, welche den Durchgang des Schafts (4) gestattet, vorsteht, und dadurch, dass das Mittel für den Druck der starren Hülse (8) gegen die deformierbare Hülse (9) von einer Buchse (3) aus Kunststoff gebildet ist, die um die Aufnahmehülse (10) geschraubt wird.

4. Einrichtung nach irgendeinem der Ansprüche 1 bis 3, dadurch gekennzeichnet, dass das Stromversorgungselement (7) in dem Gehäuse (11) untergebracht ist, wo es über der Erregerwicklung angeordnet ist, die die Form eines Solenoids (5) hat und auf der Basis (6) des Abschlusses des Gehäuses ruht, und dadurch, dass das Gehäuse (11) eine Verschlusskappe (2) hat, die ebenfalls aus nichtmagnetischem Material ist, welche um das obere Ende des Gehäuses geschraubt wird und mit einer mittigen Öffnung versehen ist, die die Montage des Unterbrechers des Erregerkreises, der von einem Steuerschalter (1) gebildet ist, gestattet.

5. Einrichtung nach irgendeinem der Ansprüche 1 bis 4, dadurch gekennzeichnet, dass der Schaft (4) abnehmbar und sterilisierbar, insbesondere durch Autoklaven-Behandlung, ist.

6. Einrichtung nach irgendeinem der Ansprüche 1 bis 5, dadurch gekennzeichnet, dass der Schaft (4) in Abhängigkeit von seinem Durchmesser austauschbar ist.

7. Einrichtung nach Anspruch 6, dadurch gekennzeichnet, dass der Schaft (4) einen oberen Teil von konstantem Durchmesser hat, der dazu bestimmt ist, von dem Solenoid (5) und der Aufnahmehülse (10) ummantelt zu sein, und einen unteren Teil von einem Durchmesser, der in reziproker Funktion von der vorhersehbaren Konzentration der pathogenen Keime des zu analysierenden Mediums variable ist.

8. Einrichtung nach Anspruch 4, dadurch gekennzeichnet, dass das Erregersolenoid (5) direkt auf den Schaft aus Weicheisen (4) gewickelt ist.

9. Einrichtung nach Anspruch 4, dadurch gekennzeichnet, dass das Erregersolenoid (5) um den Schaft (4) mittels eines festen Trägers, der insbesondere von einer Hülse (14) aus nichtmagnetischem Material gebildet ist, die leicht mit Kraft um den Schaft (4) aufgebracht ist, gewickelt ist.

## Claims

1. A magnetic device for withdrawing magnetic gell particles or the like from a biological or other liquid medium and for transferring them into an immunoenzymatic test medium, said device being of the type comprising:

a rod (4) of ferromagnetic material, in particular soft iron, and of appropriate length;

a housing (11), preferably oblong, made of a non-magnetic material, and in particular a plastic material;

an excitation circuit (1-5-7-13) for electromagnetically exciting the rod (4) and comprising, in turn:

an electrical excitation winding received in said housing (11) and wound round the rod (4);

a discontinuous core of ferromagnetic material constituted by said rod (4) whose top portion is received in the housing while its bottom portion extends outside the housing;

a power supply source for the excitation circuit, in particular constituted by an electric battery; and

a control switch (1) for controlling the excitation circuit;

said device being characterized in that it is provided with means for fixing and centering the rod (4) by clamping.

2. A device according to claim 1, characterized in that the means for centering and fixing the rod (4) by clamping are constituted by a deformable sleeve (9), in particular a rubber sleeve, and by a rigid sleeve (8) for pressing against said deformable sleeve, which sleeves are disposed about the rod and co-operate with housing means in which they are disposed end-to-end, together with means for compressing the rigid sleeve (8) against the deformable sleeve (9) whose deformation serves to clamp against the rod (4) and thus to fix it and center it relative to the housing (11).

3. A device according to claim 2, characterized in that the housing means for receiving the rigid and deformable sleeves (8, 9) is constituted by a sleeve (10) coaxial with the housing (11) and integrally molded therewith, which housing sleeve (10) projects outwardly and downwardly from the base (6) closing the housing and is pierced by a central orifice for passing the rod (4), and in that the means for compressing the rigid sleeve (8) against the deformable sleeve (9) are constituted by a ring of plastic material which screws around the housing sleeve (10).

4. A device according to any one of claims 1 to 3, characterized in that the power supply battery (7) is received in the housing (11) in which it is disposed above the excitation winding which is in the form of a solenoid (5) resting on the base (6) closing the housing, and in that said housing (11) includes a closure cap (2) also made of nonmagnetic material which screws onto the top end of the housing and which is provided with a central orifice through which the switch of the excitation circuit is mounted, said switch being constituted by a control pushbutton (1).

5. A device according to any one of claims 1 to 4, characterized in that the rod (4) is removeable and sterilizable, in particular in an autoclave.

6. A device according to any one of claims 1 to 5, characterized in that the rod (4) is interchangeable as a function of its diameter.

7. A device according to claim 6, characterized in that the rod (4) has a top portion of constant diameter for being received by said solenoid (5) and said housing sleeve (10), and a bottom portion of varying diameter with said diameter varying as an inverse function of the expected concentration of pathogenic germs in the medium to be analysed.

8. A device according to claim 4, characterized in that the excitation solenoid (5) is wound directly onto the soft iron rod (4).

9. A device according to claim 4, characterized in that the excitation solenoid (5) is wound around the rod (4) via a solid former, constituted in particular by a sleeve of non-magnetic material which is a push fit around the rod (4).

EP 0 181 798 B1

1 / 1

FIG. 1

FIG. 2

7